Europäisches Patentamt

European Patent Office

Office européen des brevets

(11) Publication number: **0 255 229**
**A2**

# (12) EUROPEAN PATENT APPLICATION

(21) Application number: 87305640.2

(22) Date of filing: 24.06.87

(51) Int. Cl.4: **G01N 31/22** , C12Q 1/22

The title of the invention has been amended (Guidelines for Examination in the EPO, A-III, 7.3).

(30) Priority: 30.06.86 US 880612

(43) Date of publication of application:
03.02.88 Bulletin 88/05

(84) Designated Contracting States:
AT BE CH DE ES FR GB GR IT LI LU NL SE

(71) Applicant: SURGICOT, INC.
55 Kennedy Drive
Smithtown New York(US)

(72) Inventor: Buglino, Steven T.
22 Lotus Road
Bayport New York(US)
Inventor: Lanniciello, Marie
16 Forest Drive
East Northport New York(US)

(74) Representative: Thomas, Roger Tamlyn et al
D. Young & Co. 10 Staple Inn
London WC1V 7RD(GB)

(54) Steam sterilisation test pack.

(57) The steam test pack is comprised of a plastic tube having a sterilization sensitive ink imprinted indicator card and a biological indicator therein. The ends of the plastic tube are capped by plastic caps having an aperature extending therethrough, and having an inner diameter slightly larger than the outer diameter of the tube. This combination creates the inhibiting menas to control the flow or exchange of a gaseous medium into the tube and then to the biological indicator.

EP 0 255 229 A2

## STEAM TEST PACK

The present invention relates to an improved test pack for testing whether a steam sterilization procedure was effective. In particular, the present invention may be used in the type of steam sterilizers which are used in hospital sterilization procedures.

Steam sterilization is a very common and frequent method of preparing sterile goods for use in health care facilities. A common method of testing for the efficacy of the steam sterilization procerss is to include a biological indicator in the load being sterilized. A biological indicator is typically comprised of a self-contained unit including a suspension of large number of bacterial spores that has been dried on a carrier, e.g., paper, and then inserted into a package, such as a glassine envelope or a plastic vial. The spore suspension, being comprised of a species that is very resistant to steam (e.g. Bacillus stearothermophilus) and present on the carrier in large numbers, acts as an indicator for the effectiveness of the steam sterilization process. If large numbers of a very steam- resistant organism are placed in a load, and if the sterilization process kills this large number of resistant spores, then this is evidence of the effectiveness of the steam sterilization process.

Since most items that are being steam sterilized are held in some sort of packaging which allows maintenance of sterility of the pack contents until the time of use, it is also prudent to enclose the biological indicator inside some type of packaging so as to equal the challenge of the sterilized packs for the biological indicator.

In order to standardize the packaging challenge, the Association for the Advancement of Medical Instrumentation (AAMI) has recommended a challenge pack in which the biological indicator as well as a chemical indicator can be positioned. However, the making of a steam biological indicator test pack is a tedious affair, in that it is laborious to construct. Other problems with making up test packs are that not all the necessary materials may be present at an institution, and no two people will construct the pack with the same density, as there is presently no standardized method of construction. Once constructed, such standard test packs continue to have problems, in that they are bulky, and they consume a large amount of volume in a chamber (space which could be used for items needing sterilization).

In order to minimize the above problems, a small biological indicator test pack which yields the same rate of survival/death of a biological indicator as does the AAMI-described test pack has been designed. The preferred test pack of the invention includes the following beneficial features:

1. It is fully disposable (no need to stockpile pack materials for reuse).

2. It is small does not use large volumes of space in a chamber).

3. It is of standardized construction (no variability from pack to pack).

4. It is already preloaded with a biological indicator (no labor is required for assembly).

5. Its behavior is equivalent to an AAMI- described biological indicator test pack in both gravity (250°F) and prevacuum (272°F) steam sterilization cycles.

In the Drawing:

FIG. 1 is a perspective view of a preferred steam test pack of the present invention; and

FIG. 2 is a cross-sectional view of the steam test pack of FIG. 1.

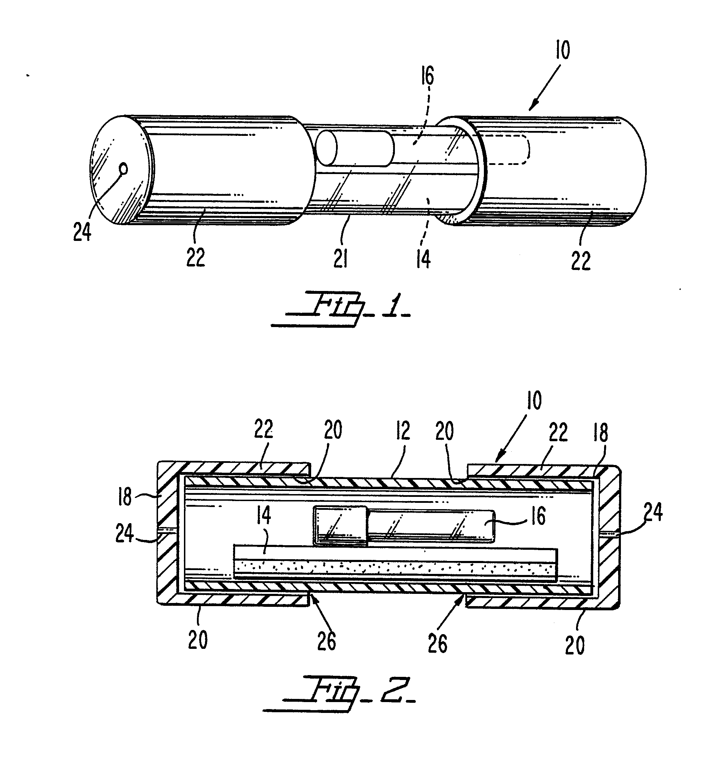

Referring generally to FIGS. 1 and 2, a test pack 10 of the present invention is shown. The test pack 10 is constructed of a clear plastics tube 12. In the illustrated embodiment of the invention, the tube 12 12 is a chemical indicator 14 comprising an indicator card which has a sterilization sensitive ink imprinted thereon and a biological indicator 16 (which may be comprised of either a glassine- enclosed spore strip or a self-contained biological indicator).

The open ends 18 of the tube 12 are restricted by barriers. In the illustrated embodiment of the invention, the barriers are comprised of polypropylene caps 22 that have aperatures 24 in their centers and inside diameters 20 slightly larger than the outside diameter of the plastic tube 12. The caps 22 have inner splines which hold them in place via a friction fit on the tubes.

In the illustrated embodiment of the invention, the closure caps 22 are made slightly larger in inner diameter than the outer diameter 18 of the tube 12 so they act as a means of metering sterilant entry. The apertures in the polypropylene caps 22 are comprised of holes 24 having diameters of 1/8", and the caps 22 have inner diameters 20 of about 1 1/32" when the outside diameter of the tube is about 1", thereby leaving vent openings 26, between the outside of the tube 12 and the inside of the caps 22. However, these dimensions and materials may be varied without departing from the scope of the present invention. Many other dimensions and materials, in various combinations, can produce the desired effect which is to allow a spore kill rate inside the pack 10 that is equivalent to that obtained in the AAMI-described pack.

In particular, by varying the weight or volume of the main tube 12, the sizes of the apertures 24, the size of the closure caps 22 and the sizes of the vent openings 26, the desired kill rate can be controlled.

The closure caps 22 remain fastened to the tube 10 by any suitable means, in particular, inner splines (not shown) are used in preferred embodiment in order to securely retain the caps 22 on the tube 12. They remain in this position even after rapid pressure changes that occur during prevacuum steam sterilization cycles. The closures caps 22 can be further fastened to the tube 12 by means of an adhesive-backed label that would extend over both the surface of the tube 12 and the surface of the closure caps 22. Such a label could also be proof of non-tampering with the disposable test pack 10, since, if the label were torn, it would evidence an attempt to open the pack 10.

The steam test pack has been described to include closure cap means 22 which provide two openings into the tube 12, namely a circuitous vent opening 26 between the inner diameter of the cap 22 of the tube 12 and a hole 24 through the cap 22. It has been found that without the two openings, 24 and 26, the steam test pack does not operate notwithstanding the fact that the opening 24 is made considerably larger than 1/8". In addition, it has been found that the illustrated test pack works properly only when it is placed in a horizontal position, i.e., when the long axis through the center of the tube is horizontal.

## Claims

1. A steam sterilization test pack comprising:
   (a) a plastics tube which is open on both ends;
   (b) a biological indicator within said tube;
   (c) an indicator card imprinted with a sterilization sensitive ink within said tube;
   (d) means for inhibiting the flow of a gaseous medium into the open ends of said tube; and
   (e) cap closure means over the ends of said tube having said flow inhibiting means, there being at least two openings for admitting or exchanging a gaseous medium through said cap closure means

2. The steam sterilization test pack of Claim 1 wherein said biological indicator is a self-contained biological indicator.

3. The steam sterilization test pack of Claim 2 wherein said plastic tube is clear and has an outer diameter of about 1", and said ink imprint on said indicator card is visible from outside said tube.

4. The steam sterilization test pack of Claim 3 wherein said means for inhibiting the flow of gaseous medium comprises a hole and a dimensional difference between the outer diameter of the tube and the inner diameter of the cap closure means.

5. The steam sterilization test pack of Claim 4 wherein said cap closure means is selected to have an inner diameter of about 1/32".

6. The steam sterilization test pack of Claim 5 wherein said openings in said cap closures comprise holes having a diameter of about 1/8".

10

16

24 22 21 14 22

_Fig_1_

10

22 20 12 20 22 18

18

24 14 16 24

20 26 26 20

_Fig_2_